(19) **Europäisches Patentamt European Patent Office Office européen des brevets**

(11) **EP 3 025 732 B1**

(12) **EUROPEAN PATENT SPECIFICATION**

(45) Date of publication and mention of the grant of the patent:
**27.03.2019 Bulletin 2019/13**

(21) Application number: **15425102.9**

(22) Date of filing: **25.11.2015**

(51) Int Cl.:
*A61K 9/127* (2006.01)     *A61K 47/36* (2006.01)
*A61K 8/55* (2006.01)      *A61K 8/73* (2006.01)
*A61Q 19/00* (2006.01)     *A61K 8/14* (2006.01)
*A61K 8/35* (2006.01)      *A61K 38/16* (2006.01)
*A61K 8/64* (2006.01)      *A61Q 19/08* (2006.01)
*A61K 31/12* (2006.01)     *A61K 31/196* (2006.01)

(54) **HYALUROSOMES, THEIR USE IN TOPICAL COSMETIC OR PHARMACEUTICAL COMPOSITIONS AND THEIR PREPARATION PROCESS**

HYALUROSOME, DEREN VERWENDUNG IN TOPISCHEN KOSMETISCHEN ODER PHARMAZEUTISCHEN ZUSAMMENSETZUNGEN UND DEREN HERSTELLUNGSVERFAHREN

HYALUROSOMES, LEUR UTILISATION DANS DES COMPOSITIONS COSMÉTIQUES TOPIQUES OU PHARMACEUTIQUES ET LEUR PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2014 IT RM20140687**

(43) Date of publication of application:
**01.06.2016 Bulletin 2016/22**

(73) Proprietor: **ICNODERM SRL**
**09123 Cagliari (IT)**

(72) Inventors:
 • **Manconi, Maria**
  **09123 Cagliari (IT)**
 • **Zaru, Marco**
  **09123 Cagliari (IT)**
 • **Castangia, Ines**
  **09123 Cagliari (IT)**
 • **Cabras, Alba**
  **09123 Cagliari (IT)**
 • **Cappai, Nadia**
  **09123 Cagliari (IT)**

 • **Fadda, Anna Maria**
  **09123 Cagliari (IT)**
 • **Manca, Maria Letizia**
  **09123 Cagliari (IT)**

(74) Representative: **Bianchetti Bracco Minoja S.r.l.**
**Via Plinio, 63**
**20129 Milano (IT)**

(56) References cited:
  **WO-A1-00/18371        FR-A1- 2 939 317**
  **US-A1- 2001 005 501   US-A1- 2008 145 415**

 • **DATABASE WPI Week 201422 Thomson Scientific, London, GB; AN 2014-F00795 XP002741833, -& CN 103 520 007 A (TIANBO MEDICAL TECHNOLOGY SUZHOU CO LTD) 22 January 2014 (2014-01-22)**
 • **BROWN M B ET AL: "THE EFFECT OF HYALURONAN ON THE IN VITRO DEPOSITION OF DICLOFENAC WITHIN THE SKIN", INTERNATIONAL JOURNAL OF TISSUE REACTIONS, BIOSCIENCE EDIPRINT, GENEVA, CH, vol. 17, no. 4, 1 January 1995 (1995-01-01), pages 133-140, XP008054051, ISSN: 0250-0868**

**Description**

This invention concerns hyalurosomes, their use in topical cosmetic or pharmaceutical compositions and their preparation process. More specifically, this invention concerns nanometric vesicles similar to liposomes (hyalurosomes) comprising molecules of hyaluronate, phospholipid molecules and at least one active pharmaceutical or cosmetic ingredient. The invention also concerns the use of hyalurosomes in topical cosmetic or pharmaceutical compositions and their preparation process.

Traditionally, the application of drugs on the skin is aimed at obtaining a softening or protective effect, or introducing active ingredients in the top skin layers. Modern pharmaceutical technology re-evaluates the cutaneous administration of drugs in view of reaching a systemic effect or targeted to the deep, underlying tissues, through the development of an accurate formulation design. In fact, using an appropriate formulation can improve the bio-pharmaceutical performance of certain drugs and/or active cosmetic substances and simplify patient compliance. This application method has been neglected for a long time due to the objective difficulty that active ingredients encounter in seeping into the skin, to reach the underlying tissues or systemic circulation.

Currently, one of the most used strategies to improve drug accumulation in the tissues below the skin is to use nano-vesicular carriers, such as liposomes and liposome-like structures conceptually derived from them, but with performance and/or functional characteristics that are more enhanced than the original system, particularly when it comes to skin delivery. Among the most used lamellar nano-vesicles are niosomes, etiosomes, transfersomes and glycerosomes. These systems are actually able to prolong the permanence of active ingredients in the skin, increase bio-availability and reduce local and systemic toxicity. These vesicles, especially transfersomes, are not only excipients that simply facilitate passage of the drug into the skin, interacting with the lipids of the stratum corneum, but are real "delivery systems" capable of carrying the drug through the stratum corneum and epidermis, all the way to the dermis. In the above-mentioned systems, which represent the latest evolution of liposome-like systems intended for topical delivery, it can be pointed out that the improvement of characteristics such as flexibility, loading capacity and stability is never synergistic, resulting only in a unilateral and partial improvement of overall performance.

It is therefore clear that there is a need to have new topical administration systems that overcome the drawbacks of known systems.

According to this invention, nano-bio-vesicles can be now obtained that have both better loading capacities than known vesicular systems and a greater ability to "retain" the encapsulated active ingredient longer within the vesicular structure, compared to the other known vesicular systems mentioned above. The vesicles in this invention have a superior "loading and retaining" capacity, both when it comes to hydrophilic and lipophilic drugs. These characteristics are reflected naturally in the performance of the vesicles on the skin. In fact, as observed in "in vitro" experiments, they are able to increase the accumulation of active ingredient carried onto the skin in vitro and, consequently, as demonstrated by "in vivo" tests, increase the therapeutic or pseudo-therapeutic biological effectiveness. The improved effectiveness of therapeutic or biological activity in vivo is clearly linked to the greater "loading and retaining" capacity of the active molecule in vesicles, coupled with the inherent and greater ability of hyalurosomes as skin delivery, due to the synergistic action of sodium hyaluronate and phospholipid molecules. In fact, hyaluronate behaves both as an absorption promoter and as a promoter of solvation, and the phospholipid molecules as carriers.

The nano-vesicles of the invention are characterised by an inter-lamellar system made up of hyaluronate and phospholipid molecules. More specifically, the nano-vesicles of this invention do not have molecules of hyaluronate on the surface only, i.e. they are not liposomes coated on the surface with molecules of hyaluronate, like certain known liposomes (U.S. patent US6890901 deposited by Marriot et al.), but consist of an inter-lamellar system where the molecules of hyaluronic acid are also within the phospholipid bilayer (double layer) of these nano-vesicles. As such, the nano-vesicles of this invention are hybrid inter-lamellar systems of hyaluronate and phospholipid molecules similar to liposomes, which can be referred to as hyalurosomes. The nano-vesicles of this invention also contain an active ingredient that is incorporated in this inter-lamellar system. The structure of hyalurosomes according to this invention is the result of a new method of preparation which, unlike known methods for the preparation of liposomes, allows the hyaluronate chains to interact with the phospholipid portions that are more akin in aqueous solvent, thus creating a kind of interaction that is maintained in the vesicular structuring and creating a more solvating power that comes in handy when introducing insoluble active ingredients in aqueous media.

Known methods for the preparation of liposomes, in fact, include the solubilisation of phospholipids in organic solvents that are subsequently made to evaporate (US2008145415; CN103520007). The result of these known methods is a lipid film notes from which subsequent hydration gives origin to phospholipid bilayers that lead to the formation of liposomes. Again, according to the above-mentioned known methods, hyaluronic acid solutions are added after the formation of the lipid film. The hyaluronic acid, therefore, does not participate in the formation of the inter-lamellar structure with the phospholipid vesicles, but can move only superficially or into the hydrophilic core of the liposomes. In light of the above, the known methods do not concern the preparation of hyalurosomes.

FR2939317 discloses topical cosmetic compositions comprising niosomes or liposomes with lipid bilayers, comprising hyaluronic acid inside the vesicles. The liposomes can be prepared by introducing the lipid mixture under agitation in an aqueous solution comprising hyaluronic acid at elevated temperature. CN103520007 discloses liposomes which contain hyaluronic acid and which are prepared by the lipid film method, wherein a hyaluronic acid solution is added to the lipid film which is previously prepared by mixing the phospholipid and the organic solvent.

The inter-lamellar hybrid structure of hyalurosomes, according to this invention, is prepared through a process in which an aqueous dispersion of active ingredient and hyaluronate is used to hydrate the phospholipid molecules at a room temperature of approximately 20-28°C, and in the absence of organic solvents. Therefore, thanks to this invention process, hyaluronate can interact with the more akin phospholipid portions in aqueous media, thus creating a kind of interaction that is maintained in the vesicular structure, and thus becoming an integral part of the structure of nano-vesicles, and not just a surface of coating of the vesicles. The structure of the hyalurosomes in this invention allows to obtain the following advantages:

- increased solvation of the encapsulated agent, which triggers an effect on the dissolution kinetics and consequently a greater capacity of the active ingredient (especially if poorly soluble or insoluble) to enter the solution in biological liquids;
- increased loading capacity and vesicular stability compared to known vesicular systems or liposomal systems that are mixed with hyaluronic acid after forming the liposome (U.S. patent US6890901 deposited by Marriot et al.);
- increased capacity to retain the active ingredient for longer, even when the ingredients are hydrophilic substances, because these are previously captured in the structured vehicle formed by the sodium hyaluronate in water, which then bonds with the phospholipids and remains anchored to them;
- increased "skin enhancement" capacity compared to other topical delivery systems of vesicular nature, due to the synergistic action of sodium hyaluronate with phospholipid vesicles.

The specific subject of this invention, therefore, includes the process as defined in claim 1. The subject of the present invention also includes liposome-like vesicles (hyalurosomes) for topical pharmaceutical or cosmetic application, obtainable by the process disclosed in this invention and comprising: a) hyaluronate, preferably sodium hyaluronate, b) at least one phospholipid and c) at least one active pharmaceutical or cosmetic ingredient, wherein said at least one phospholipid forms a double layer in which said hyaluronate is arranged, as defined in claim 9. Moreover, the hyaluronate is also found in the aqueous core and on the external surface of the vesicles, besides inside the double layer.

The invention further relates to pharmaceutical or cosmetic compositions for topical use comprising said vesicles, to said vesicles or compositions for use in the medical treatment of skin disorders, and to the non-medical use of said vesicles or compositions in the cosmetic treatment of skin disorders.

A topical pharmaceutical or cosmetic application means that the vesicles of the invention are intended for topical applications in the pharmaceutical, cosmetic and Medical Devices industry. The vesicles of this invention are specific to the topical pharmaceutical or cosmetic application, in particular to the application on both healthy and injured skin, and on mucous membranes.

Hyaluronate molecules, for example are molecules such as sodium hyaluronate or other hyaluronic acid salts that are soluble in water, in which hyaluronic acid has a molecular weight of 0.05 to 10 Kda, preferably 0.2 to 5 Kda, and more preferably 0.4 to 2 Kda.

In addition, these vesicles do not contain organic solvents such as Chloroform, Dichloromethane and Ethanol.

Phospholipids that can be used according to this invention, for instance, include one or more natural or synthetic phospholipids, pure or mixed, such as soy or egg lecithin, hydrogenated or non-hydrogenated phosphatidylcholine in varying degrees of purity, phosphatidylethanolamine, phosphatidylserine, phosphatidylglycerol, phosphatidylinositol, dimyristoylphosphatidylcholine (DMPC), dipalmitoyl phosphatidylcholine (DPPC), distearoylphosphatidylcholine, palmitoyl-stearoylphosphatidylcholine, sphingomyelin, mixtures of hydrogenated and non-hydrogenated phospholipids derived from soybeans, preferably hydrogenated and non-hydrogenated soybean phosphatidylcholine.

The essential characteristics of phospholipids selected for the preparation of hyalurosomes are the ability to form lamellar vesicles, the high biocompatibility, the low metabolic degradation and the absence of toxicity.

The nano-bio-vesicles (hyalurosomes) of this invention are very versatile and can be used as vehicles of active hydrophilic, lipophilic and amphiphilic agents. The first will be completely dissolved within the watery cavities of the vesicles, i.e. the central core and the watery thin layers between the double layers, together with the hyaluronate polymer, which is therefore able to better capture and retain the hydrophilic molecules that remain caught in the midst of its chains. For this reason, these vesicles, unlike liposomes, have a high efficiency of encapsulation, even with hydrophilic drugs. Lipophilic or amphipathic drugs, instead, will be in part interlayered between the chains of the phospholipid in the bilayer and in part, permanently dispersed in the aqueous solution of sodium hyaluronate, thanks to molecular interactions with the polymer.

The active pharmaceutical and cosmetic ingredients to be used according to this invention include antivirals, vitamins such as vitamin C and vitamin E, plant extracts, anti-inflammatory drugs, corticosteroids, antibiotics, fungicides, anti-psoriasis drugs, acyclovir, trans-retinoic acid, betamethasone, lidocaine, minoxidil, amphotericin B, gentamicin, rifampicin, vitamin E and its esters, diclofenac, lidocaine hydrochloride, alkaloids, hydroxy acids, antioxidants and humectants. Preferably, the active pharmaceutical and cosmetic ingredients are for topical use.

Therefore, according to this invention, it is possible to use both lipophilic compounds such as curcumin, quercetin, resveratrol, campherol, diclofenac, acyclovir, trans-retinoic acid, betamethasone dipropionate, minoxidil, gentamicin, rifampicin, tocopherol and its derivatives (esters) or lipophilic derivatives of vitamin E, and hydrophilic compounds like, for example, phycocyanin, caffeic acid, caffeine, diclofenac sodium, lidocaine hydrochloride, vitamin C and its derivatives, and soluble derivatives of tocopherol.

The nano-vesicles according to this invention may include also non-ionic surfactants that are compatible with the formation of vesicles (usually used in the preparation of niosomes) or co-solvents and/or modifiers.

The modifiers are added to the formulation as stabilizers and/or as inducers of the electric charge of vesicles, and can be, for example, cholesterol, stearylamine, oleic acid, dicetylphosphate and phosphatidylglycerol (PG), preferably cholesterol or phosphatidylglycerol. Surfactants or aqueous co-solvents are used to increase the solubility of the drug and can be, for example, glycerol, propylene glycol, ethylene glycol, isopropyl alcohol, polyethylene glycols, polysorbates, poly-glucosidic ethers, preferably glycerol or propylene glycol.

In vesicles according to this invention, said hyaluronate (a) has a concentration of 0.01% to 5%, preferably 0.02% to 2%, more preferably 0.1% to 1% and said at least one phospholipid (b) has a concentration of 0.1% to 30%, preferably of 0.5% to 25%, more preferably of 1% to 15%; in which said percentages are by weight of hyaluronate or phospholipid compared to the weight of vesicular dispersion.

By way of example and without any limitation of the object of the present patent, the hyalurosomes can have the composition shown in table 1 below:

Table 1

| Phospholipid component | Sodium hyaluronate | Modifiers | Non-ionic surfactants or co-solvents | Active ingredient | Preservatives | H$_2$O |
|---|---|---|---|---|---|---|
| 1-30% | 0.05-2% | 0-2% | 5-50% | 0-5 % | 0-1% | as required to 100% |

Depending on their composition, the hyalurosomes of this invention may appear in the form of unilamellar lipid vesicles (small or large) or oligo- or multi-lamellar lipid vesicles. The hyalurosomes can have variable dimensions, though always within the nanometre range, with a mean diameter of between 50 and 500 nm.

Moreover, the hyalurosomes of this invention may be incorporated in formulations intended for topical application, such as gels, lotions, emulsions, suspensions and the like.

Therefore, it a further object of the present invention a pharmaceutical or cosmetic composition for topical use comprising the nano-vesicles of this invention, in combination with one or more of the pharmaceutically or cosmetically acceptable excipients or adjuvants.

This invention also concerns the nano-vesicles or compositions described above, for use in the treatment of skin disorders. Treatments can be both cosmetic and medical. Among the medical treatments, the treatment of scar alterations and/or preventive and curative treatment of keloid; the treatment of seborrheic and atopic dermatitis; the symptomatic treatment of some autoimmune diseases like psoriasis can be listed. Examples of cosmetic treatments based on the use of vesicles according to this invention are treatments with anti-aging function, with antioxidant function, draining effect, moisturising and nourishing function, or with soothing post-peeling function.

The vesicles of this invention (hyalurosomes), thanks to the synergic action of phospholipids and of sodium hyaluronate, have a greater loading and skin-delivery capacity than liposomes which, of themselves, generally favour the accumulation of drugs in the skin compared to conventional topical formulations such as creams, lotions, ointments or solutions.

When the hyalurosomes of this invention are applied to the skin, some of the vesicles, thanks to the synergic action of their constituents, phospholipids and sodium hyaluronate, act as enhancers and probably merge with the lamellar inter-corneocyte matrix, and fluidify it. Then, the other vesicles that have remained intact seep through the structure more easily and reach the epidermis and dermis, or accumulate in the stratum corneum, forming a deposit system. Therefore, the vesicles according to this invention have the advantage of simply acting as promoters of skin absorption and as active carriers of drugs, by enhancing local bioavailability and the therapeutic or biological activity of the molecules that carry onto the skin.

Furthermore, it is a further object of the present invention a process for the preparation of said vesicles of hyalurosomes comprising the following steps: a) preparing an aqueous solution or dispersion of at least one active pharmaceutical or cosmetic ingredient in an aqueous solution of hyaluronate; b) mixing the solution or dispersion of step a) with at least one phospholipid until hydration of said phospholipid and the formation of a colloidal system comprising the vesicles of this invention. Both step a) and step b) are conducted at temperatures below 30°C, preferably 20 to 28°C, preferably at about 25°C. Therefore, the process according to this invention does not require the use of organic solvents and the formation of lipid films.

According to the process of this invention, the concentration of hyaluronate in the aqueous solution of hyaluronate, preferably sodium hyaluronate, to which the active ingredient is added, may vary from 0.01% to 5%, preferably from 0.02% to 2%, more preferably from 0.1% to 1%. The active agent is added to this solution in a concentration of 0.1% (w/v) to 5% (w/v), preferably 1% (w/v).

Said at least one phospholipid can be used in concentrations ranging from 0.1% to 30%, preferably from 0.5% to 25%, more preferably from 1% to 15% w/w, the percentages being by phospholipid weight compared to the weight of the dispersion.

As mentioned above, the hyaluronate molecules, for example, are molecules of sodium hyaluronate or other hyaluronic acid salts that are soluble in water, in which hyaluronic acid has a molecular weight from 0.05 to 10 Kda, preferably from 0.2 to 5 Kda, and more preferably from 0.4 to 2 Kda.

Phospholipids that can be used according to this invention, for instance, include one or more natural or synthetic phospholipids, pure or mixed, such as soy or egg lecithin, hydrogenated or non-hydrogenated phosphatidylcholine in varying degrees of purity, phosphatidylethanolamine, phosphatidylserine, phosphatidylglycerol, phosphatidylinositol, dimyristoylphosphatidylcholine (DMPC), dipalmitoyl phosphatidylcholine (DPPC), distearoylphosphatidylcholine, palmitoyl-stearoylphosphatidylcholine, sphingomyelin, mixtures of hydrogenated and non-hydrogenated phospholipids derived from soybeans, preferably hydrogenated and non-hydrogenated soybean phosphatidylcholine.

The active pharmaceutical and cosmetic ingredients to be used according to this invention include antivirals, vitamins such as vitamin C and vitamin E, plant extracts, anti-inflammatory drugs, corticosteroids, antibiotics, fungicides, anti-psoriasis drugs, acyclovir, trans-retinoic acid, betamethasone, lidocaine, minoxidil, amphotericin B, gentamicin, rifampicin, vitamin E and its esters, diclofenac, lidocaine hydrochloride, alkaloids, hydroxy acids, antioxidants and humectants. Preferably, the active pharmaceutical and cosmetic ingredients are for topical use.

The process according to this invention may include also non-ionic surfactants that are compatible with the formation of vesicles (usually, the surfactants used in the preparation of niosomes are used) or co-solvents and/or modifiers. These surfactants or co-solvents and/or modifiers are added in the lipophilic or aqueous phase, depending on their solubility characteristics. For example, they can be added together with the active ingredient, because they are usually selected based on the characteristics of solubility of the active ingredient.

As described above, the modifiers are added to the formulation as stabilizers and/or as inducers of the electric charge of vesicles, and can be, for example, cholesterol, stearylamine, oleic acid, dicetyl phosphate and phosphatidylglycerol (PG), preferably cholesterol or phosphatidylglycerol.

Surfactants or aqueous co-solvents are used to increase the solubility of the drug and can be, for example, glycerol, propylene glycol, ethylene glycol, ethyl alcohol, isopropyl alcohol, polyethylene glycols, polysorbates, polyglucosidic ethers, preferably glycerine, propylene glycol.

The aqueous co-solvent or a surfactant can be used in a concentration of 10% (v/v) to 30% (v/v) and preferably to 20% (v/v), in which the percentage represents the volume of surfactant or co-solvent compared to the volume of vesicular dispersion.

The modifier can be used in concentrations ranging from 0.01% (w/w) to 2% (w/w), preferably from 0.05% (w/w) to 1% (w/w) or more preferably from 0.1% (w/w) to 0.5% (w/w).

The process according to this invention may further comprise a step c) of sonication. Sonication can be carried out, for example, through 10 to 60 cycles (every cycle on for 5 seconds and off for 2 seconds), with an amplitude of 14-15 or preferably from 15 to 50 cycles (every cycle on for 5 seconds and off for 2 seconds), with an amplitude of 14-15 or more preferably from 20 to 40 cycles (every cycle on for 5 seconds and off for 2 seconds) with an amplitude of 14-15.

According to a particular embodiment of this invention, the process for the preparation of hyalurosomes consists in the preparation of an aqueous solution of sodium hyaluronate in a concentration between 0.05% (w/v) and 2% (w/v) and preferably 0.1% (w/v), to which the active agent is added, in a concentration of 0.1% (w/v) to 5% (w/v), preferably 1% (w/v). The dispersion is left for 5 hours under constant agitation at temperatures between 20 and 28°C, preferably at 25°C, in order to finely disperse the molecules of the active ingredient that interact with the polymer chains and remain suspended within the viscoelastic network formed by the sodium hyaluronate in the aqueous solution. Subsequently, the various phospholipids (pure or mixed, hydrogenated and non-hydrogenated, among which the mixtures of soy and egg phosphatidylcholine, in varying degrees of purity and containing saturated and/or unsaturated phospholipids) are hydrated with the aqueous dispersion containing the active ingredient dispersed in the sodium hyaluronate. The dispersion is then again left for 5 hours under constant agitation at a room temperature between 20 and 28°C, preferably at 25°C, thus allowing the slow hydration of the phospholipids, and is then sonicated.

As stated above, the process of this invention, compared to the known processes for the preparation of other vesicular systems, does not require the use of organic solvents, normally used to solubilise and then dry the lipids in the form of a film. In fact, in this invention, the drug is dispersed in a solution of sodium hyaluronate and the phospholipids are hydrated by direct addition of this dispersion, therefore leaving the system under agitation for 5 hours. This ensures that phospholipids swell slowly and spontaneously form lamellar vesicles in which the hyaluronate chains that have interacted with the phospholipid portions most similar to them can be more easily incorporated within the phospholipid bilayer. Moreover, the presence of a strong polyelectrolyte like sodium hyaluronate, which interacts electrostatically with them, besides contributing to the process of hydration of phospholipids, even those that have a transition temperature of more than 30°C, cause a solvating action capable of favouring and facilitating the dissolution of insoluble active ingredients.

If the active agent is not homogeneously dispersed in the aqueous solution of sodium hyaluronate, or if a phospholipid with a transition temperature above 30°C is used, an aqueous co-solvent or compatible non-ionic surfactant (like the one used to prepare niosomal systems), in a concentration between 10% (v/v) and 30% (v/v) and preferably 20% (v/v), can be added, to improve the solubilisation of the active ingredient and/or swelling of the phospholipid. In fact, the presence of a compatible non-ionic surfactant or aqueous co-solvent in the hydration step has a synergistic effect with that of the sodium hyaluronate, and facilitates the solvation of the molecules and therefore, also the swelling of the phospholipids. In particular, when the transition temperature of the phospholipids is higher than 30°C, they do not moisturise spontaneously in water at room temperature, whereas they hydrate in the same conditions, in the presence of surfactants or co-solvents.

To the aqueous solution of drug and sodium hyaluronate aqueous co-solvents such as glycerol, ethylene glycol, propylene glycol, Transcutol and/or non-ionic surfactants that are compatible with a vesicular system, hydrophiles that are soluble in water, such as PEG of different molecular weight, tween, oramixCG 110 can be possibly added.

The process according to this invention allows to obtain vesicles having better characteristics when compared to other known carrier systems. In particular, the inclusion of the hyaluronate polymer at a certain step of preparation process triggers a set of dynamics, immediately noticeable through the morphological and dimensional parameters (small vesicles 150 nm and highly homogeneous PI = 0.26), which are reflected in terms of performance of the vesicles, i.e. increased solvation and dissolution kinetics of the lipophilic agents, resulting in increased loading and topical delivery capacity, and stability, differentiating them. The vesicles according to this invention therefore are clearly distinguishable from other known vesicular systems covered by hydrophilic polymers.

Moreover, the preparation process used for this invention has the added benefit of allowing all the phospholipids to hydrate, without the aid of organic solvents and at a temperature below 30°C, preferably ≈25°C, even those whose transition temperature is above 30°C, without the need to heat them above their transition temperature, as it occurs in the preparation of the known lamellar vesicles; and of facilitating the solvation of the active ingredients and of capturing them in the polymer network, stabilising aqueous dispersion.

Whichever phospholipid is used, even if its transition temperature is more than 25°C, the hyalurosomes are prepared at a temperature below 30°C (about 25°C), while for the preparation of conventional liposomes, the temperature is greater than or equal to the transition temperature of the phospholipid used. For example, the liposomes of hydrogenated phosphatidylcholine are usually prepared at 60°C and those of DPPC at 45°C. In fact, if the hydrogenated phosphatidylcholine is left in water, under agitation, at 25°C for 12 hours, it does not swell, does not form double layers, and remains on the bottom in the form of powder. Instead, using a mixture of sodium hyaluronate to hydrate the hydrogenated phosphatidylcholine and leaving the dispersion under agitation at 25°C for 5 hours, a homogeneous dispersion similar to a diluted gel is obtained, in which the phosphatidylcholine is fully hydrated, thanks to the presence of hyaluronic acid, a polyelectrolyte that interacts electrostatically with phosphatidylcholine, which acts as a humectant and facilitates the swelling in water. The fact that there is no need to heat the dispersion of hyalurosomes represents a clear advantage if the active ingredient to be incorporated is thermolabile or partially degradable, when subjected to heating.

Subsequently, if the vesicles are larger than 400 nm, the dispersion is sonicated to reduce the size of the vesicles.

This forms hybrid lamellae with alternating double layers consisting of polymer thin layers, in which the negatively charged carboxylic groups of polymer molecules of hyaluronate interact electrostatically with the positive portion of the polar heads of the phospholipids, and remain anchored to these, forming a continuous system where the molecules of the active agent are trapped between the polymer chains or in the double layer. As such, the hyaluronate will strengthen the inter-lamellar packing, forming a hybrid system between the phospholipid and polymer in which the lipid bilayer is in a condition of limited mobility of the phospholipids in the midst of the polymeric network and the active agent remains caught and protected in this hybrid system.

Other substances (cholesterol, charged lipids, etc.) can be added to the basic system, as described above, which have a purely adjuvant action that therefore is not restrictive to its performance, unlike other topical delivery systems of vesicular nature, known to the state of the art (Vemuri S, Rhodes CT. 1995; Cevc G. 2004; Torchilin VP, Weissig V, 2003, glycerosomes 2010).

This invention will now be described by way of non-limiting example, according to its preferred embodiments, with particular reference to the figures of the attached drawings, wherein:

**Figure 1.** Cryo-TEM image of hyalurosomes incorporating 1% curcumin and prepared with sodium hyaluronate in concentrations of 0.05% (A) and 0.1% (B) .

**Figure 2.** Amount of accumulated curcumin (%) in the stratum corneum (SC), in the epidermis (Ep), dermis (D) and receptor compartment (CR) after 24 hours of experiment using Franz cells. The experiments were repeated at least six times (no.=6) for each sample, and the values were reported as average values $\pm$ standard deviation, the ° symbol indicates that the values are different (w<0.05) from those of the PEG400/water dispersion used as control sample, while the # symbol indicates that the values are equal to those of the control sample.

**Figure 3.** Images of ulcerated rats with TPA (A) and treated with the dispersion of curcumin in PEG400/water (B), with liposomes incorporating curcumin (C), with the hyalurosomes incorporating curcumin and prepared with sodium hyaluronate in concentrations of 0.05% (D) and 0.1% (E).

**Figure 4.** Images of the skin cut into thin perpendicular strips on the skin's surface. The phycocyanin has been coloured in blue and the phospholipids in red.

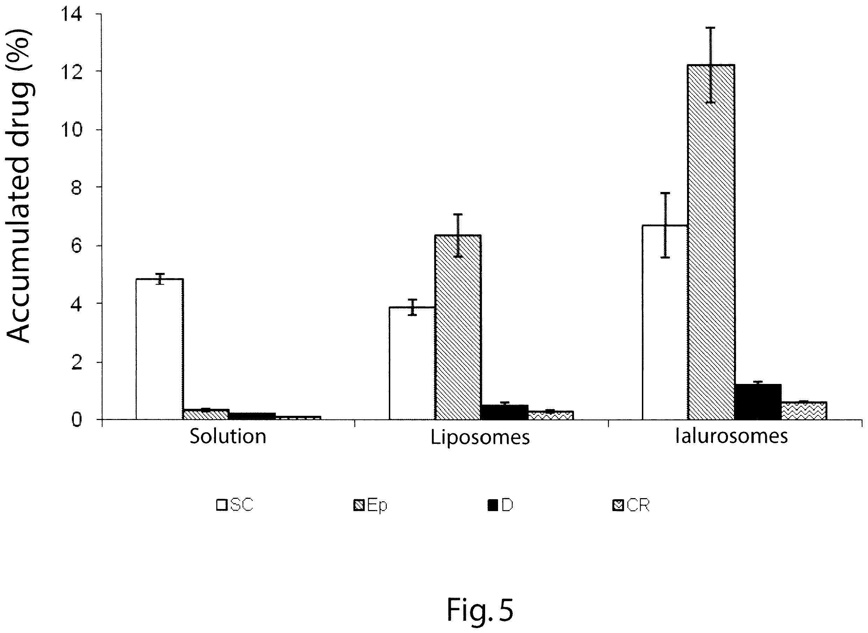

**Figure 5.** Amount of accumulated diclofenac acid (%) in the stratum corneum (SC), in the epidermis (Ep), dermis (D) and receptor compartment (CR) after 24 hours of experiment using Franz cells. The experiments were repeated at least six times (no.=6) for each sample and the values were reported as average values $\pm$ standard deviation.

**EXAMPLE 1:** *Preparation of hyalurosomes according to the invention and containing curcumin, diclofenac or phycocyanin and physical-chemical characterisation of the hyalurosomes.*

To highlight the potential and characteristics of the hyalurosomes without limiting their purpose, an antioxidant and anti-inflammatory of natural origin (curcumin), or a powerful, synthetic non-steroidal anti-inflammatory drug (diclofenac) in acid form, were incorporated into these vesicles as lipophilic drugs. Alternatively, phycocyanin, which is a natural phycoprotein with anti-inflammatory and antioxidant properties, was encapsulated as hydrophilic drug. Given the high loading capacity of hyalurosomes, each of these drugs was loaded into the vesicles in high concentrations and traditional liposomes were prepared to be used as reference. The chemical and physical characteristics and functions of hyalurosomes were then compared with those of conventional liposomes.

MATERIALS AND METHODS

### 1.1 Preparation of hyalurosomes and liposomes

Table 2 shows the percentage composition of the preparations of hyalurosomes incorporating curcumin in concentrations of 0.5% and 1% (0.5CUR and 1CUR).

Table 2

|  | P90G (%) | Sodium hyaluronate (%) | Curcumin (%) |
|---|---|---|---|
| 0.05 Hyalurosomes 0.5CUR | 18 | 0.05 | 0.5 |
| 0.05 Hyalurosomes 1CUR | 18 | 0.1 | 1.0 |
| 0.1 Hyalurosomes 0.5CUR | 18 | 0.05 | 0.5 |
| 0.1 Hyalurosomes 1CUR | 18 | 0.1 | 1.0 |

The hyalurosomes incorporating 0.5% curcumin were prepared by weighing 0.5 grams of curcumin and 0.05 grams of sodium hyaluronate (sample 0.05Hyal-0.5CUR) or 0.1 grams of sodium hyaluronate (sample 0.1Hyal-0.5 CUR) and placed in 81.45 (sample 0.05Hyal-0.5CUR) or 81.40 (sample 0.1Hyal-0.5CUR) grams of water and left to stir for 5 hours at 25$\pm$3°C. 18 grams of Phospholipon 90 G (p90 G) was added to the dispersion obtained for both samples and left them to stir for 5 hours at 25°C. The samples thus obtained were then sonicated until a homogeneous dispersion was obtained. The size of the vesicles was monitored and, if necessary, the hyalurosomes were sonicated (30 cycles of 2 seconds with breaks of 5 seconds) with an ultrasonic disintegrator.

The reference liposomes were prepared using the classical method of lipid film, obtained by dissolving the lipophilic components in a solvent (chloroform or ethanol) and then removing the solvent by means of a rotary evaporator. Subsequently, the aqueous hydration solution was added to the lipophilic components (phospholipids, additives and curcumin), distributed in the form of a thin film on the walls of the container. The dispersion obtained was left to stir for 12 hours at a temperature higher than the transition temperature of the phospholipid used (e.g. 45°C for the DPPC; 25°C for the phosphatidylcholine).

**Table 3.** Percentage composition of the preparations of hyalurosomes and liposomes encapsulating 2% phycocyanin.

| | P90G (%) | Sodium hyaluronate (%) | PEG400 (%) | Phycocyanin (%) |
|---|---|---|---|---|
| 0.1 Hyalurosomes PEG 400.1 | 6 | 0.1 | | 2 |
| Hyalurosomes | 18 | 0.1 | 10 | 2 |
| Liposomes | 18 | | | 2 |

The hyalurosomes incorporating 2% phycocyanin were prepared by weighing 2 grams of phycocyanin and 0.1 grams of sodium hyaluronate (sample 0.1Hyal) or 2 grams of phycocyanin, 0.1 grams of sodium hyaluronate and 10 grams of PEG 400 (sample 0.1 Hyal-PEG 400) and placed in 91.9 (sample 0.1 Hyal) or 71.90 (sample 0.1 Hyal-PEG 400) grams of water and left to stir for 5 hours at $25\pm3°C$. 6 grams of Phospholipon 90 G (p90 G) was added to the dispersion obtained for the sample 0.1 Hyal and 18 grams of Phospholipon 90 G (p90 G) for the sample 0.1 Hyal-PEG 400 and left to stir for 5 hours at 25°C. The samples thus obtained were then sonicated until a homogeneous dispersion was obtained. The size of the vesicles was monitored and, if necessary, the hyalurosomes were sonicated (30 cycles of 2 seconds with breaks of 5 seconds) with an ultrasonic disintegrator.

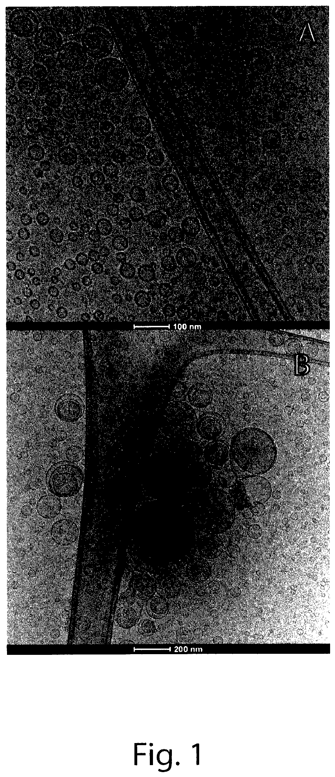

The actual formation of hyalurosomes incorporating curcumin was confirmed under the optical microscope with polarised light, which showed the Maltese crosses, indicating the presence of a lamellar system [Manosroi A et al., 2003; Mele S et al., 2003; Sinico C et al 2005]. The formation of closed, single, oligo- or multi-lamellar vesicles was confirmed under the traditional transmission electron (TEM) and cryogenic (cryo-TEM) microscopes, as shown in Figure 1. The samples containing 0.05% sodium hyaluronate are predominantly unilamellar, while those containing 1% are formed both by vesicles by unilamellar and multi-lamellar vesicles.

## 1.2 Characterisation of hyalurosomes and liposomes

The characterisation of hyalurosomes was carried out by observing the samples under an optical microscope with polarised light and a transmission electron microscope at low temperature (cryo-TEM), dimensionally analysing the zeta potential using Photon Correlation Spectroscopy (PCS) and measuring the encapsulation efficiency (E%).

To prepare the samples for observation under the cryo-TEM, a drop of dispersion was made to absorb on a carbon grid, the excess was absorbed with filter paper, and the grid was vitrified by immersing it in ethane maintained at melting point. The sample was observed with a Tecnai F20 TEM (FEI Company) and photographed at 200 kV, -170/175°C, using a CCD Eagle camera (FEI Company).

The average size and dimensional distribution amplitude (PI) were measured with the aid of Photon Correlation Spectroscopy, using the Zetasizer nano-ZS (Malvern Instrument, UK) while the Z potential was measured using the same Zetasizer nano-ZS, with the aid of Phase Analysis Light Scattering (M3-PALS), which measures the electrophoretic mobility of the particles in a temperature-controlled room.

The incorporation efficiency (for lipophilic drugs) and encapsulation efficiency (for hydrophilic drugs) expresses the percentage of drug actually encapsulated in the vesicles, and then recovered after purification, compared to the initial amount (100%) found at the end of preparation. The vesicular dispersion was separated from the free drug by dialysis. The dispersion (1-2 ml) was transferred in a dialysis tube sealed at both ends, and was immersed in a water bath, maintained at 25°C and under constant stirring. The amount of water bath must be sufficient to solubilise all the drug inside the dialysis tube; if necessary, the water can be changed 1 or 2 times, so as to be sure to keep out all the free drug. The vesicular dispersions before and after dialysis were diluted with methanol (1/1000), thus bursting the vesicles and freeing the drug, and the drug concentration was quantified by HPLC Analysis.

## 1.3 Quantitative determination of curcumin and diclofenac

The quantitative determination of curcumin was carried out with an HPLC, Alliance 2690 (Waters, Milan, Italy), with Spectrophotometric UV detector, wavelength of 427 nm, using a SunFire C18 column (3.5 $\mu$m, 4.6x150 mm) and a mixture of acetonitrile, water and acetic acid for the mobile phase (94.8:5:0.2, v/v) eluted at a speed of 1 ml min$^{-1}$.

The quantitative determination of diclofenac was carried out with the same instrument, at 227 nm, a SunFire C18 column (3.5 $\mu$m, 4.6x150 mm) and a mixture of acetonitrile and water for the mobile phase (70:30, v/v) eluted at a speed of 0.5 ml min$^{-1}$.

The quantitative determination of phycocyanin was carried out using a UV/visible spectrophotometer calibrated at 615 nm. To accurately determine the amount of phycocyanin, a calibration line was first built with 5 solutions of phycocyanin in water with known titre.

*1.4 Average diameter, polydispersity index (PI), zeta potential and encapsulation efficiency (E%) of hyalurosomes*

The incorporation efficiency of an active ingredient expresses the ratio between the amount of drug actually incorporated inside of vesicles and the one in the dispersion. The drug not incorporated in the vesicles was removed from the dispersion by dialysis (biblio que cur). The amount of active ingredient contained in the dispersions was quantified under the HPLC; to release the drug incorporated inside these vesicles, the latter were burst by adding a large amount of methanol (1/1000) capable of solubilising all the phospholipid used.

Taking into account the initial amount of drug and the amount found after dialysis, the incorporation efficiency (E%) expressed as a percentage was calculated according to the formula:

$$E\% = amount\ of\ active\ ingredient\ in\ purified\ dispersion$$
$$x\ 100/amount\ of\ active\ ingredient\ in\ initial\ dispersion$$

Using 18% of the phospholipid, all the systems prepared are able to carry the curcumin both in concentrations of 0.5% and 1%, with good encapsulation efficiencies, which are always higher in the case of hyalurosomes (Table 4). The hyalurosomes confirm their greater loading capacity; in fact, they are able to incorporate approximately 79% of curcumin, while the liposomes only ~66%. Moreover, in the liposomal dispersion incorporating 1% curcumin, immediately after preparation, a precipitate is always observed on the bottom of the test tube, which can be dispersed immediately by simply shaking the tube.

**Table 4.** Average diameter, polydispersity index (PI), zeta potential and efficiency of incorporation (E%) of hyalurosomes and liposomes incorporating curcumin in concentrations of 0.5% and 1% (0.5CUR and 1CUR). The values were measured on the day of preparation and the table lists the average values $\pm$ standard deviation obtained from at least six samples (no.=6).

| | Average diameter (nm) | PI | Zeta potential (mV) | E (%) |
|---|---|---|---|---|
| 0.05 Hyalurosomes 0.5 CUR | 151$\pm$10 | 0.28 | -26$\pm$7 | 82$\pm$6 |
| 0.05 Hyalurosomes 1 CUR | 166$\pm$9 | 0.30 | -26$\pm$4 | 76$\pm$5 |
| 0.1 Hyalurosomes 0.5 CUR | 163$\pm$10 | 0.29 | -25$\pm$5 | 81$\pm$7 |
| 0.1 Hyalurosomes 1 CUR | 177$\pm$12 | 0.30 | -24$\pm$6 | 79$\pm$4 |
| Liposomes 0.5 CUR | 192$\pm$11 | 0.42 | -29$\pm$6 | 69$\pm$5 |
| Liposomes 1 CUR | 196$\pm$18 | 0.46 | -30$\pm$5 | 63$\pm$4 |

The main physical and chemical properties of hyalurosomes, i.e. average diameter, poly-dispersity index and zeta potential were measured using a Dynamic Laser Light Scattering (Table 4), the day of preparation and then every week for 4 weeks, so as to monitor the stability of the system. The method of preparation of hyalurosomes allows to obtain small vesicles (approximately 150 nm) and poorly poly-dispersed (PI~0.26). Moreover, the dispersions obtained are particularly stable over time; during the 90 days of study, there is no precipitate formation and the size of their particles remain constant. When the vesicles, instead, are prepared according to the method conventionally used to obtain liposomes coated with a polymer (i.e. when the liposomes containing the drug are first prepared and then the polymer is added), the average size is larger (about 220 nm), the index of dispersion is always greater than 0.32 and above all, after 2-3 weeks, the drug precipitates and two separate phases form in the dispersion. Probably because of the larger size, the coated formulations allow an accumulation of the drug in the skin that is comparable to that of conventional liposomes and the aqueous solution of the drug. The hyalurosomes according to this invention have characteristics that are very similar those of the reference liposomes, but are slightly smaller and less poly-dispersed, the average size is between 151 nm and 177 nm, the dispersity index is always $\leq$ 0.30, which indicates a good dispersion homogeneity, the value of zeta potential is highly negative, and this ensures a good system stability, because it prevents the aggregation of vesicles in dispersion. The size of the hyalurosomes and the efficiency of incorporation of the curcumin remain constant during the 4 weeks, while the size of the liposomes grows up to 280 nm, and their incorporation efficiency lowers by as much as ~50%.

The characteristics of the vesicles containing phycocyanin are listed in Table 5.

Table 5. Average diameter, polydispersity index (PI), zeta potential and encapsulation efficiency (E%) of liposomes and hyalurosomes encapsulating phycocyanin (1%). The table shows the average values ± standard deviation (no.=3).

| Samples | Average (nm) | PI | Zeta potential (mV) | E (%) |
|---|---|---|---|---|
| 0.1 Hyalurosomes | 130±4 | 0.3 0 | -18±7 | 49± 8 |
| PEG 0.1 Hyalurosomes | 135±4 | 0.2 9 | -10±4 | 45± 7 |
| Liposomes | 145±8 | 0.4 3 | -20±9 | 34± 6 |

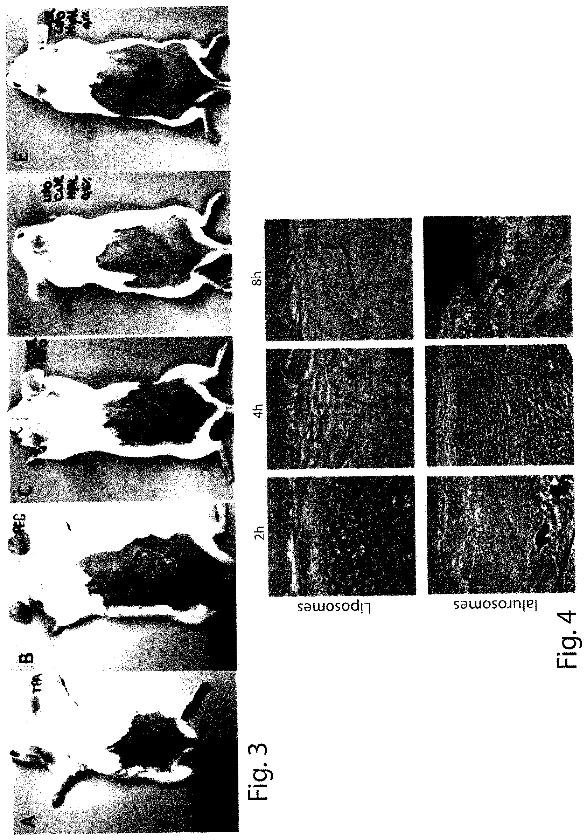

The phycocyanin was encapsulated in the hyalurosomes, with and without PEG400, with greater efficiency than that obtained with the reference liposomes, about 34% in the first and 49% in the second. This confirms the greater loading capacity of hyalurosomes, even with hydrophilic drugs, thanks to the formation of the structured carrier formed by the sodium hyaluronate, which remains anchored to the lipid bilayer. In addition, the hyalurosomes are statistically smaller than the liposomes and are less polydispersed, so that as in the case of the hyalurosomes incorporating the curcumin, even using a hydrophilic drug with high molecular weight, the chemical and physical characteristics of the hyalurosomes are more promising than those of liposomes. To test the ability of the vesicles to carry the drug through the skin, the vesicles were marked with a fluorescent phospholipid, phosphatidylethanolamine-dioleoyl-sulforhodamine (Rho-PE), and the distribution in the skin of phycocyanin, which is fluorescent (blue), and of the phospholipids (red) was observed at different stages, using a confocal microscope. To better simulate the patho-physiological conditions of superficial wounds or skin lesions, the stratum corneum was removed from the pig skin by stripping and the phycocyanin formulations were applied directly on the live skin. The images of the skin (Fig. 3) indicate that the phycocyanin carried in the hyalurosomes penetrates easily and quickly.

As mentioned above, to demonstrate the greater capacity of hyalurosomes as skin carriers, a powerful synthetic non-steroidal anti-inflammatory drug was encapsulated, 1% diclofenac in acid form. The physical and chemical characteristics of the hyalurosomes and their corresponding liposomes are listed in Table 6.

Table 6. Average diameter, polydispersity index (PI), zeta potential and encapsulation efficiency (E%) of liposomes and hyalurosomes encapsulating diclofenac acid (1%). The table shows the average values ± standard deviation (no. =3).

| Samples | Average (nm) | PI | Zeta potential (mV) | E (%) |
|---|---|---|---|---|
| 0.1 Hyalurosomes | 142±11 | 0.30 | -20±7 | 85±7 |
| Liposomes | 187±7 | 0.40 | -18±5 | 74±5 |

In this case, too, the hyalurosomes are smaller, are less polydispersed and incorporate a higher quantity of drug compared to the reference liposomes. Their better characteristics, such as drug delivery, actually translate into a greater accumulation of the drug in the skin, especially in the stratum corneum and dermis, compared to what obtained with the solution and reference liposomes.

**EXAMPLE 2:** *In vitro study of cutaneous penetration/permeation*

In vitro studies were conducted using a baby pig's skin, removed immediately after the death of the animal, washed with water, laid out, wrapped in aluminium foils and then frozen at -80°C. At the time of the experiment, the skin was cut into discs and interposed between the two compartments of the Franz cells, having an effective area of diffusion of 0.785 cm$^2$. The receptor cell was filled with a saline solution (5.5 ml of 0.9% NaCl) and was kept under constant agitation. In the donor compartment, 40 μL of each sample was introduced, at start and after 4 hours, taking care to cover the entire surface of the skin. For each formulation, six cells were used. The cells were kept at a controlled temperature of 37°C (so that the skin temperature is equal to the physiological temperature of 32°C). At predetermined times (2, 4, 6, 8 and 24 hours), the receptor compartment was emptied and refilled with fresh saline solution. The sampled solutions were freeze-dried, then resumed with 2 ml of methanol and analysed under the HPLC.

At the 24th hour, the skin was carefully washed, dried with filter paper and separated into individual layers: stratum corneum, epidermis and dermis. The stratum corneum was separated by *stripping* with Master-Aid RollTex tape. The tape was applied to the skin for 10 seconds, applying slight pressure, and then removed. After each strip (minimum 4 times), the strips of tape used were cut into small pieces and put into a test tube with 3 ml of methanol to extract the drug. The dermis was separated from the skin with a scalpel, cut into small pieces and transferred into a test tube with 3 ml of methanol. The methanolic solutions were sonicated (2 cycles of one minute each) with a Soniprep 150 (MSE Crowley, UK), so as to extract the drug from the skin. The samples were analysed under the HPLC to determine the amount of drug.

RESULTS

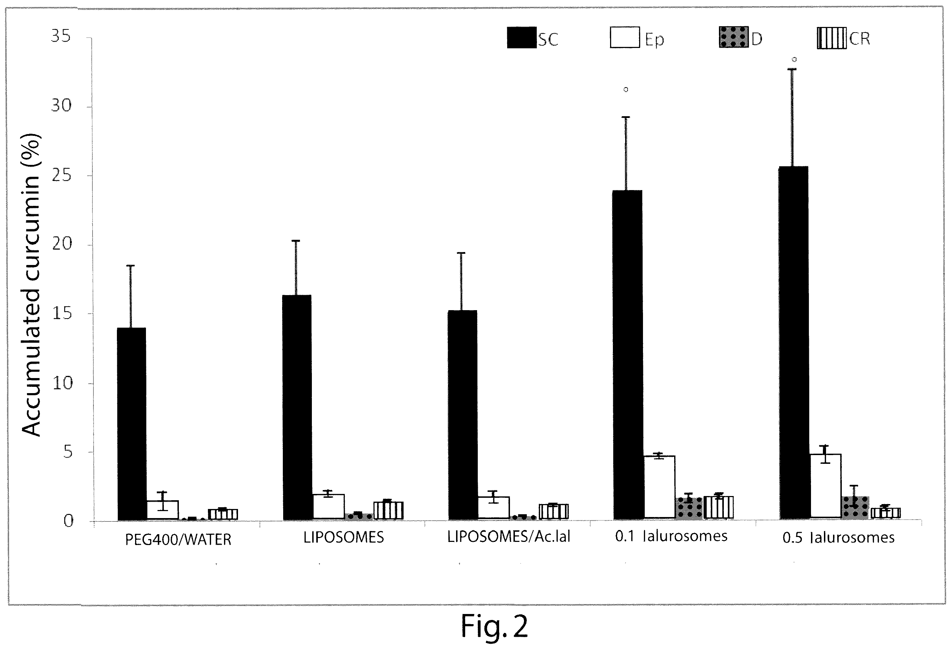

The ability of the vesicles to interact with the skin and facilitate the transition of active substances, through the stratum corneum and epidermis, down to the dermis or underlying tissues, was tested through in vitro experiments to measure cutaneous penetration, using curcumin, which is a lipophilic molecule that alone has a hard time penetrating into the skin. As a comparison, to measure the effectiveness of hyalurosomes as skin carriers, liposomes were used and a dispersion of curcumin in PEG400 and water (5%). The studies were conducted using the highest concentration of curcumin (1%), and the hyalurosomes were prepared with a concentration of 0.05% and 0.1% of sodium hyaluronate (Figure 2).

The results reported in Figure 2 clearly highlight the greater capacity of hyalurosomes, compared with the liposomes and dispersion, in acting as carriers and facilitating the accumulation of curcumin, especially in the stratum corneum, but also in the epidermis and dermis, regardless of the amount of sodium hyaluronate used. In all cases, even when applying it to the dispersion, the accumulation of curcumin is always much higher than in the stratum corneum and progressively decreases in the other layers.

**EXAMPLE 3:** *Cutaneous distribution of phycocyanin and phospholipids*

The vesicles encapsulating phycocyanin were prepared by adding Rho-PE (0.35 mg/ml) to the phospholipids during preparation. The marked vesicles were applied in vitro on the skin, as shown in example 4. At the end, the pieces of skin were cut into thin slices (25 $\mu$m), at right angles to the skin surface, with a cryo-microtome (Leica CM1950, Barcelona, Spain). They were then observed with an inverted confocal microscope FluoView files (Olympus, Barcelona, Spain), using an UPlanSApo lens 20$\times$ 0.75 NA. Images were obtained of the size 1024$\times$1024 $\mu$m. The phycocyanin and Rho-PE were excited, 600 nm and 559 nm respectively, measured at 640 nm and 578 nm.

**EXAMPLE 4:** *Study of in vivo inhibition of myeloperoxidase and edema*

Female mice Hsd were used for the in vivo experiments: ICR (CD-1®) 5-6 weeks old (weight 25-35 g) bought from Harlan Laboratories (Barcelona, Spain). The mice were given 1 week to acclimatise before use. All studies were performed in accordance with the rules of the European Union for the management and use of laboratory animals. The protocols have been approved by the Institutional Committee for the care and use of animals of the University of Valencia. The backs of the mice were shaved and were applied ($\sim$2 cm$^2$) of 12-or-tetradecanoylphorbol-13-acetate (TPA) dissolved in acetone (243 $\mu$M; 3 $\mu$g/ 20 $\mu$l) to induce skin inflammation and ulceration (day 1). The control mice were applied only acetone (20 $\mu$l). The dispersion of curcumin or vesicles incorporating curcumin were applied (40 $\mu$l) drop by drop 3 and 6 hours after application of the TPA. The process was repeated (every 24 hours) on days 2 and 3. On day 4, the mice were sacrificed by cervical dislocation. Each group consisted of four mice. Inhibition of inflammation and ulcers were quantified by measuring the formation of edema and myeloperoxidase (MPO). The dorsal skin area treated was removed and weighed to evaluate any increase indicative of edema formation. For the measurement of the MPO, skin biopsies were homogenised and centrifuged, and the supernatant was incubated with hydrogen peroxide and tetramethyl benzidine. MPO activity was quantified at 620 nm spectroscopically. The degree of ulceration was assessed visually.

RESULTS

The studies have shown that compared to liposomes, hyalurosomes allow for a high accumulation of the two fluorescent markers in the stratum corneum, and especially over a longer time span, they favour a discreet build-up in the lower layers, the epidermis and dermis. The presence of a high concentration of vesicles in the outermost layer of the skin is probably linked to their ability to interact with the stratum corneum, within which they then merge with intercellular lipids. The hyalurosomes are able to disrupt the orderly and complex structure of the most superficial layer of the skin, but they are also capable of increasing the degree of hydration of the stratum corneum, in both cases favouring the accumulation of the drug in the skin.

As regards the curcumin, the tests confirm the greater capacity of hyalurosomes of this invention to carry the curcumin in the skin and to increase their bioavailability in skin tissues, using in vivo tests to measure the effectiveness of curcumin, free or nano-encapsulated, in treating skin ulcers.

**Table 7.** Values of edema, inhibition of edema (edema I), myeloperoxidase (MPO) and inhibition of myeloperoxidase (MPO I) in healthy mice, ulcerated with TPA and treated with curcumin (CUR) dispersed in PEG400/water or incorporated into liposomes and hyalurosomes. The table shows the average values $\pm$ standard deviation (no.=6).

| Treatment | Edema (mg) | Edema I (%) | MPO (ng/mg) | MPO I (%) |
|---|---|---|---|---|
| Healthy mice | 3.9±0.38 | 100 | 57±17 | 100 |
| TPA | 10.7±0.68 | 0 | 362±19 | 0 |
| CUR PEG400 | 7.1±0.36 | 53±2 | 298±19 | 20±2 |
| CUR Liposomes | 5.2±0.60 | 80±3 | 156±21 | 67±3 |
| CUR 0.05 Hyalurosomes | 4.8±0.75 | 86±4 | 108±22 | 83±4 |
| CUR 0.1 Hyalurosomes | 5.4±0.53 | 81±2 | 111±18 | 82±3 |

To induce an injury in mice, tetradecanoylphorbol acetate (TPA), a highly irritating and necrotising substance, was used which if applied repeatedly on the skin, causes ulceration and inflammation with the formation of edema, infiltration of neutrophils and an increase of myeloperoxidase (MPO), an enzyme that indicates inflammation. The application of TPA for 3 consecutive days actually causes an ulceration of the skin and edema formation (Figure 3 A); treatment of the wound with the dispersion of curcumin in PEG400/water leads only to a slight improvement of the lesion, which appears to be drier and more desquamated. The treatment with nano-vesicular curcumin (liposomes and hyalurosomes) always leads to an improvement of the overall conditions of the wound, where the skin tissue has partially (liposomes) or almost completely (hyalurosomes) reformed and appears pinkish and flexible like the one of the non-ulcerated skin. In mice treated with liposomes, the skin lesion is reduced in intensity and extension, but the outline of the skin tissue is not totally reformed and there are still signs of ulcerated areas. While in mice treated with hyalurosomes, especially those containing 0.1% sodium hyaluronate, the skin looks completely reformed and pinkish, with some desquamation in the mice treated with 0.05 hyalurosomes. Even the values of edema and MPO in mice treated with liposomes and hyalurosomes are reduced compared to those of mice ulcerated with TPA and non-treated or treated with dispersion in PEG400/water, and as such, the corresponding inhibition values increase considerably. In particular, the improvement is most noticeable in mice treated with 0.1 hyalurosomes, in which the inhibition of edema reaches approximately 84% and that of MPO about 83%.

Therefore, the greater loading and skin-delivery capacity of hyalurosomes translates into the improved therapeutic effectiveness of the drug, probably also due to the synergistic effect of sodium hyaluronate, which restructures the epidermal tissue and is used as an active ingredient in the treatment of skin lesions.

### References

- "Characterization of vesicles prepared with various non-ionic surfactants mixed with cholesterol"; Aranya Manosroi a,b,*, Paveena Wongtrakul c, Jiradej Manosroi a,b, Hideki Sakai d,e, Fumio Sugawara d, Makoto Yuasa d,e, Masahiko Abe d,e, Received 6 February 2003; accepted 14 March 2003.
- "Liposomes as carriers for dermal delivery of tretinoin: in vitro evaluation of drug permeation and vesicle-skin interaction" C. Sinico, M. Manconi, M. Peppi, D. Valenti, F. Lai, A.M. Fadda. Journal of Controlled Release,103, 123-136 (2005).
- "Quantitative characterization of phospholipids in milk fat via 31P NMR using a monophasic solvent mixture". Lipids. 2003 May;38(5):585-91; Murgia S, Mele S, Monduzzi M.
- "Lipid vesicles and other colloids as drug carriers on the skin" Adv Drug Deliv Rev. 2004 Mar 27;56(5):675-711. Cevc G
- M. Zaru, M.L. Manca, A.M. Fadda, G.Orsini (2008), "GLYCEROSOMES AND USE THEREOF IN PHARMACEUTICAL AND COSMETIC PREPARATIONS FOR TOPICAL APPLICATION", patent application WO 2010/102770;
- Manca, M. L., Zaru, M., Manconi, M., Lai, F., Valenti, D., Sinico, C., Fadda, A. M. (2013). Glycerosomes: A new tool for effective dermal and transdermal drug delivery. International Journal of Pharmaceutics, 455 (1-2), 66-74.

### Claims

**1.** A process for the preparation of nano-metric liposome-like vesicles (hyalurosomes) for topical pharmaceutical or cosmetic application comprising a) hyaluronate, preferably sodium hyaluronate, b) at least one phospholipid and c) at least one active pharmaceutical or cosmetic ingredient, wherein said at least one phospholipid forms a double layer and said hyaluronate is disposed inside the double layer itself,
said process comprising the following steps:

> a) preparing an aqueous solution or dispersion of at least one active pharmaceutical or cosmetic ingredient in an aqueous solution of hyaluronate;
> b) mixing the solution or dispersion of step a) with at least one phospholipid until hydration of at least one phospholipid and the formation of a colloidal system comprising the vesicles,

wherein the process does not comprise the use of an organic solvent and wherein both step a) and step b) are conducted at temperatures below 30°C, preferably 20 to 28°C, even more preferably at about 25°C.

2. Process according to claim 1, wherein the concentration of hyaluronate in the aqueous solution of hyaluronate, preferably sodium hyaluronate, to which the active ingredient is added, ranges from 0.01% (w/w) to 5% (w/w), preferably 0.02% (w/w) to 2% (w/w), more preferably 0.1% (w/w) to 1% (w/w).

3. Process according to anyone of the claims 1-2, wherein said at least one active ingredient is added to the solution of hyaluronate in a concentration of 0.1% (w/v) to 5% (w/v), preferably 1% (w/v).

4. Process according to anyone of the claims 1-3, wherein said at least one phospholipid is used in concentrations ranging from 0.1% (w/w) to 30% (w/w), preferably from 0.5% (w/w) to 25% (w/w), more preferably from 1% (w/w) to 15% (w/w) .

5. Process according to anyone of claims 1-4, wherein said at least one phospholipid is selected from the group consisting of soy or egg lecithin, hydrogenated or non-hydrogenated phosphatidylcholine in varying degrees of purity, phosphatidylethanolamine, phosphatidylserine, phosphatidylglycerol, phosphatidylinositol, dimyristoylphosphatidylcholine (DMPC), dipalmitoyl phosphatidylcholine (DPPC), distearoylphosphatidylcholine, palmitoyl-stearoylphosphatidylcholine, sphingomyelin, mixtures of hydrogenated and non-hydrogenated phospholipids derived from soybeans, preferably hydrogenated and non-hydrogenated soybean phosphatidylcholine.

6. Process according to anyone of the claims 1-5, wherein said at least one active pharmaceutical or cosmetic ingredient is selected from the group consisting of antivirals, vitamins such as vitamin C and vitamin E, plant extracts, anti-inflammatory drugs, corticosteroids, antibiotics, fungicides, anti-psoriasis drugs, acyclovir, trans-retinoic acid, betamethasone, lidocaine, minoxidil, amphotericin B, gentamicin, rifampicin, vitamin E and its esters, diclofenac, lidocaine hydrochloride, alkaloids, hydroxy acids, antioxidants and humectants.

7. Process according to anyone of the claims 1-6, which also comprises the use of non-ionic surfactants or co-solvents, and/or modifiers, wherein the modifiers are chosen from the group consisting of cholesterol, stearylamine, oleic acid, dicetylphosphate and phosphatidylglycerol (PG), preferably cholesterol and phosphatidylglycerol.

8. Process according to anyone of the claims 1-7, which further comprises a step c) of sonication.

9. Nano-metric liposome-like vesicles (hyalurosomes) for topical pharmaceutical or cosmetic application, obtainable by the process of claims 1-8, wherein said vesicles comprise: a) hyaluronate, preferably sodium hyaluronate, b) at least one phospholipid and c) at least one active pharmaceutical or cosmetic ingredient, wherein said at least one phospholipid forms a double layer and said hyaluronate is disposed inside the double layer itself.

10. Vesicles according to claim 9, wherein said at least one phospholipid is selected from the group consisting of soy or egg lecithin, hydrogenated or non-hydrogenated phosphatidylcholine, phosphatidylethanolamine, phosphatidylserine, phosphatidylglycerol, phosphatidylinositol, dimyristoylphosphatidylcholine (DMPC), dipalmitoyl phosphatidylcholine (DPPC), distearoylphosphatidylcholine, palmitoyl-stearoylphosphatidylcholine, sphingomyelin, mixtures of hydrogenated and non-hydrogenated phospholipids derived from soybeans, preferably hydrogenated and non-hydrogenated soybean phosphatidylcholine.

11. Vesicles according to anyone of the claims 9-10, wherein said at least one active pharmaceutical or cosmetic ingredient is selected from the group consisting of antivirals, vitamins such as vitamin C and vitamin E, plant extracts, anti-inflammatory drugs, corticosteroids, antibiotics, fungicides, anti-psoriasis drugs, acyclovir, trans-retinoic acid, betamethasone, lidocaine, minoxidil, amphotericin B, gentamicin, rifampicin, vitamin E and its esters, diclofenac, lidocaine hydrochloride, alkaloids, hydroxy acids, antioxidants and humectants.

**12.** Vesicles according to anyone of the claims 9-11, further comprising non-ionic surfactants or co-solvents, and/or modifiers, wherein the modifiers are chosen from the group consisting of cholesterol, stearylamine, oleic acid, dicetylphosphate and phosphatidylglycerol (PG), preferably cholesterol and phosphatidylglycerol.

**13.** Vesicles according to anyone of the claims 9-12, wherein said hyaluronate (a) has a concentration of 0.01% to 5%, preferably 0.02% to 2%, more preferably 0.1% to 1%, and said at least one phospholipid (b) has a concentration of 0.1% to 30%, preferably 0.5% to 25%, more preferably 1% to 15%; wherein said percentages are by weight of hyaluronate or phospholipid with respect to the weight of vesicular dispersion.

**14.** Pharmaceutical or cosmetic composition for topical use comprising the vesicles of claims 9-13, as the active ingredient, in combination with one or more pharmaceutically or cosmetically acceptable excipients or adjuvants.

**15.** Vesicles as defined in anyone of claims 9-13 or composition as defined in claim 14, for use in the medical treatment of skin disorders, such as atopic and seborrheic dermatitis, or psoriasis.

**16.** Non-medical use of vesicles as defined in claims 9-13 or of a composition as defined in claim 14, in the cosmetic treatment of skin disorders, such as dehydration, oxidative ageing, scar/cheloide alterations.


**Patentansprüche**

**1.** Verfahren zur Herstellung von nanoskaligen liposomartigen Vesikeln (Hyalurosomen) zur topischen pharmazeutischen oder kosmetischen Anwendung, die a) Hyaluronat, vorzugsweise Natriumhyaluronat, b) wenigstens ein Phospholipid und c) wenigstens einen pharmazeutischen oder kosmetischen Wirkstoff umfassen, wobei das wenigstens eine Phospholipid eine Doppelschicht bildet und sich das Hyaluronat innerhalb der Doppelschicht selbst befindet, wobei das Verfahren die folgenden Schritte umfasst:

a) Herstellen einer wässrigen Lösung oder Dispersion von wenigstens einem pharmazeutischen oder kosmetischen Wirkstoff in einer wässrigen Lösung von Hyaluronat;
b) Mischen der Lösung oder Dispersion von Schritt a) mit wenigstens einem Phospholipid bis zur Hydratisierung des wenigstens einen Phospholipids und bis zur Bildung eines kolloidalen Systems, das die Vesikel umfasst;

wobei das Verfahren keine Verwendung eines organischen Lösungsmittels umfasst und wobei sowohl Schritt a) als auch Schritt b) bei Temperaturen unterhalb von 30 °C, vorzugsweise bei 20 bis 28 °C, besonders bevorzugt bei etwa 25 °C, durchgeführt werden.

**2.** Verfahren gemäß Anspruch 1, wobei die Konzentration von Hyaluronat in der wässrigen Lösung von Hyaluronat, vorzugsweise Natriumhyaluronat, zu der der Wirkstoff hinzugefügt wird, im Bereich von 0,01 Gew.-% bis 5 Gew.-%, vorzugsweise 0,02 Gew.-% bis 2 Gew.-%, besonders bevorzugt 0,1 Gew.-% bis 1 Gew.-%, liegt.

**3.** Verfahren gemäß einem der Ansprüche 1-2, wobei der wenigstens eine Wirkstoff in einer Konzentration von 0,1% (w/v) bis 5% (w/v), vorzugsweise 1% (w/v), zu der Lösung von Hyaluronat hinzugefügt wird.

**4.** Verfahren gemäß einem der Ansprüche 1-3, wobei das wenigstens eine Phospholipid in Konzentrationen im Bereich von 0,1 Gew.-% bis 30 Gew.-%, vorzugsweise 0,5 Gew.-% bis 25 Gew.-%, besonders bevorzugt 1 Gew.-% bis 15 Gew.-%, verwendet wird.

**5.** Verfahren gemäß einem der Ansprüche 1-4, wobei das wenigstens eine Phospholipid aus der Gruppe ausgewählt ist, die aus Soja- oder Eilecithin, hydriertem oder unhydriertem Phosphatidylcholin in verschiedenen Reinheitsgraden, Phosphatidylethanolamin, Phosphatidylserin, Phosphatidylglycerin, Phosphatidylinosit, Dimyristoylphosphatidylcholin (DMPC), Di-palmitoylphosphatidylcholin (DPPC), Distearoylphosphatidylcholin, Palmitoylstearoylphosphatidylcholin, Sphingomyelin, Gemischen von hydrierten und unhydrierten Phospholipiden, die von Sojabohnen stammen, vorzugsweise hydriertem und unhydriertem Soja-Phosphatidylcholin, besteht.

**6.** Verfahren gemäß einem der Ansprüche 1-5, wobei der wenigstens eine pharmazeutische oder kosmetische Wirkstoff aus der Gruppe ausgewählt ist, die aus antiviralen Wirkstoffen, Vitaminen, wie Vitamin C und Vitamin E, Pflanzenextrakten, entzündungshemmenden Wirkstoffen, Corticosteroiden, Antibiotika, Fungiziden, Anti-Psoriasis-Wirkstoffen, Acyclovir, trans-Retinsäure, Betamethason, Lidocain, Minoxidil, Amphotericin B, Gentamicin, Rifampicin, Vitamin E und seinen Estern, Diclofenac, Lidocain-Hydrochlorid, Alkaloiden, Hydroxysäuren, Antioxidantien und Feuchthaltemitteln besteht.

7. Verfahren gemäß einem der Ansprüche 1-6, das außerdem die Verwendung von nichtionischen Tensiden oder Cosolventien und/oder Modifikatoren umfasst, wobei die Modifikatoren aus der Gruppe ausgewählt sind, die aus Cholesterin, Stearylamin, Ölsäure, Dicetylphosphat und Phosphatidylglycerin (PG), vorzugsweise Cholesterin und Phosphatidylglycerin, besteht.

8. Verfahren gemäß einem der Ansprüche 1-7, das weiterhin einen Schritt c) der Ultraschallbehandlung umfasst.

9. Nanoskalige liposomartige Vesikel (Hyalurosomen) zur topischen pharmazeutischen oder kosmetischen Anwendung, erhältlich durch das Verfahren gemäß den Ansprüchen 1-8, wobei die Vesikel umfassen: a) Hyaluronat, vorzugsweise Natriumhyaluronat, b) wenigstens ein Phospholipid und c) wenigstens einen pharmazeutischen oder kosmetischen Wirkstoff, wobei das wenigstens eine Phospholipid eine Doppelschicht bildet und sich das Hyaluronat innerhalb der Doppelschicht selbst befindet.

10. Vesikel gemäß Anspruch 9, wobei das wenigstens eine Phospholipid aus der Gruppe ausgewählt ist, die aus Soja- oder Eilecithin, hydriertem oder unhydriertem Phosphatidylcholin, Phosphatidylethanolamin, Phosphatidylserin, Phosphatidylglycerin, Phosphatidylinosit, Dimyristoylphosphatidylcholin (DMPC), Di-palmitoylphosphatidylcholin (DPPC), Distearoylphosphatidylcholin, Palmitoylstearoylphosphatidylcholin, Sphingomyelin, Gemischen von hydrierten und unhydrierten Phospholipiden, die von Sojabohnen stammen, vorzugsweise hydriertem und unhydriertem Soja-Phosphatidylcholin, besteht.

11. Vesikel gemäß einem der Ansprüche 9-10, wobei der wenigstens eine pharmazeutische oder kosmetische Wirkstoff aus der Gruppe ausgewählt ist, die aus antiviralen Wirkstoffen, Vitaminen, wie Vitamin C und Vitamin E, Pflanzenextrakten, entzündungshemmenden Wirkstoffen, Corticosteroiden, Antibiotika, Fungiziden, Anti-Psoriasis-Wirkstoffen, Acyclovir, trans-Retinsäure, Betamethason, Lidocain, Minoxidil, Amphotericin B, Gentamicin, Rifampicin, Vitamin E und seinen Estern, Diclofenac, Lidocain-Hydrochlorid, Alkaloiden, Hydroxysäuren, Antioxidantien und Feuchthaltemitteln besteht.

12. Vesikel gemäß einem der Ansprüche 9-11, weiterhin umfassend nichtionische Tenside oder Cosolventien und/oder Modifikatoren, wobei die Modifikatoren aus der Gruppe ausgewählt sind, die aus Cholesterin, Stearylamin, Ölsäure, Dicetylphosphat und Phosphatidylglycerin (PG), vorzugsweise Cholesterin und Phosphatidylglycerin, besteht.

13. Vesikel gemäß einem der Ansprüche 9-12, wobei das Hyaluronat (a) eine Konzentration von 0,01 bis 5%, vorzugsweise 0,02 bis 2%, besonders bevorzugt 0,1 bis 1%, aufweist und das wenigstens eine Phospholipid (b) eine Konzentration von 0,1 bis 30%, vorzugsweise 0,5 bis 25%, besonders bevorzugt 1 bis 15%, aufweist, wobei die Prozentangaben Gewichtsprozente von Hyaluronat oder Phospholipid sind, bezogen auf das Gewicht der vesikulären Dispersion.

14. Pharmazeutische oder kosmetische Zusammensetzung zur topischen Verwendung, die die Vesikel gemäß den Ansprüchen 9-13 als Wirkstoff in Kombination mit einem oder mehreren pharmazeutisch oder kosmetisch annehmbaren Arzneimittelhilfsstoffen oder Adjuvantien umfasst.

15. Vesikel gemäß einem der Ansprüche 9-13 oder Zusammensetzung gemäß Anspruch 14 zur Verwendung bei der medizinischen Behandlung von Hautstörungen, wie atopischer und seborrhoischer Dermatitis oder Psoriasis.

16. Nichtmedizinische Verwendung von Vesikeln gemäß den Ansprüchen 9-13 oder einer Zusammensetzung gemäß Anspruch 14 bei der kosmetischen Behandlung von Hautstörungen, wie Dehydrierung, oxidativer Alterung, Narben-/Keloid-Veränderungen.

**Revendications**

1. Procédé de préparation de vésicules de type liposome nano-métriques (hyalurosomes) pour une application pharmaceutique ou cosmétique topique comprenant a) du hyaluronate, de préférence du hyaluronate de sodium, b) au moins un phospholipide et c) au moins un ingrédient pharmaceutique ou cosmétique actif, dans lequel ledit au moins un phospholipide forme une double couche et ledit hyaluronate est disposé à l'intérieur de la double couche elle-même,

ledit procédé comprenant les étapes suivantes :

a) la préparation d'une solution aqueuse ou d'une dispersion d'au moins un ingrédient pharmaceutique ou cosmétique actif dans une solution aqueuse de hyaluronate,
b) le mélange de la solution ou de la dispersion de l'étape a) avec au moins, un phospholipide jusqu'à l'hydratation d'au moins un phospholipide et la formation d'un système colloïdal comprenant les vésicules,

où le procédé ne comprend pas l'utilisation d'un solvant organique et où les deux étape a) et étape b) sont effectuées à des températures au-dessous de 30 °C, de préférence de 20 à 28 °C, encore davantage de préférence à environ 25 °C.

2. Procédé selon la revendication 1, dans lequel la concentration de hyaluronate dans la solution aqueuse de hyaluronate, de préférence de hyaluronate de sodium, à laquelle l'ingrédient actif est ajouté, se situe dans la plage allant de 0,01 % (p/p) à 5 % (p/p), de préférence de 0,02 % (p/p) à 2 % (p/p), davantage de préférence de 0,1 % (p/p) à 1 % (p/p).

3. Procédé selon l'une quelconque des revendications 1 à 2, dans lequel ledit au moins un ingrédient actif est ajouté à la solution de hyaluronate à une concentration de 0,1 % (p/v) à 5 % (p/v), de préférence à 1 % (p/v).

4. Procédé selon l'une quelconque des revendications 1 à 3, dans lequel ledit au moins un phospholipide est utilisé à des concentrations se situant dans la plage allant de 0,1 % (p/p) à 30 % (p/p), de préférence de 0,5 % (p/p) à 25 % (p/p), davantage de préférence de 1 % (p/p) à 15 % (p/p).

5. Procédé selon l'une quelconque des revendications 1 à 4, dans lequel ledit au moins un phospholipide est choisi dans le groupe constitué par le soja ou la lécithine d'oeuf, la phosphatidylcholine hydrogénée ou non hydrogénée à divers degrés de pureté, la phosphatidyléthanolamine, la phosphatidylsérine, le phosphatidylglycérol, le phosphatidylinositol, la dimyristoylphosphatidylcholine (DMPC), la dipalmitoylphosphatidylcholine (DPPC), la distéaroylphosphatidylcholine, la palmitoyl-stéaroylphosphatidylcholine, la sphingomyéline, les mélanges de phospholipides hydrogénés et non hydrogénés dérivés de graines de soja, de préférence de la phosphatidylcholine de graine de soja hydrogénée et non hydrogénée.

6. Procédé selon l'une quelconque des revendications 1 à 5, dans lequel le au moins un ingrédient pharmaceutique ou cosmétique actif est choisi dans le groupe constitué par des antiviraux, des vitamines, telles que la vitamine C et la vitamine E, des extraits de plante, des médicaments anti-inflammatoires, des corticostéroïdes, des antibiotiques, des fongicides, des médicaments anti-psoriasis, l'acyclovir, l'acide trans-rétinoïque, la bétaméthasone, la lidocaïne, le minoxidil, l'amphotéricine B, la gentamicine, la rifampicine, la vitamine E et ses esters, le diclofénac, le chlorhydrate de lidocaïne, des alcaloïdes, des hydroxy acides, des antioxydants et des humectants.

7. Procédé selon l'une quelconque des revendications 1 à 6, qui comprend aussi l'utilisation de tensioactifs non ioniques ou de co-solvants, et/ou de modificateurs, où les modificateurs sont choisis dans le groupe constitué par le cholestérol, la stéarylamine, l'acide oléique, le dicétylphosphate et le phosphatidylglycérol (PG), de préférence le cholestérol et le phosphatidylglycérol.

8. Procédé selon l'une quelconque des revendications 1 à 7, qui comprend en outre une étape c) de sonication.

9. Vésicules de type liposome nano-métriques (hyalurosomes) pour une application pharmaceutique ou cosmétique topique, que l'on peut obtenir par le procédé selon les revendications 1 à 8, dans lesquelles lesdites vésicules comprennent : a) du hyaluronate, de préférence du hyaluronate de sodium, b) au moins un phospholipide et c) au moins un ingrédient pharmaceutique ou cosmétique actif, où ledit au moins un phospholipide forme une double couche et ledit hyaluronate est disposé à l'intérieur de la double couche elle-même.

10. Vésicules selon la revendication 9, dans lesquelles ledit au moins un phospholipide est choisi dans le groupe constitué par le soja ou la lécithine d'oeuf, la phosphatidylcholine hydrogénée ou non hydrogénée, la phosphatidyléthanolamine, la phosphatidylsérine, le phosphatidylglycérol, le phosphatidylinositol, la dimyristoylphosphatidylcholine (DMPC), la dipalmitoyl phosphatidylcholine (DPPC), la distéaroylphosphatidylcholine, la palmitoyl-stéaroylphosphatidylcholine, la sphingomyéline, les mélanges de phospholipides hydrogénés et non hydrogénés dérivés de graines de soja, de préférence la phosphatidylcholine de graine de soja hydrogénée et non hydrogénée.

**11.** Vésicules selon l'une quelconque des revendications 9 à 10, dans lesquelles ledit au mois un ingrédient pharmaceutique ou cosmétique actif est choisi dans le groupe constitué par des antiviraux, des vitamines, telles que la vitamine C et la vitamine E, des extraits de plante, des médicaments anti-inflammatoires, des corticostéroïdes, des antibiotiques, des fongicides, des médicaments anti-psoriasis, l'acyclovir, l'acide trans-rétinoïque, la bétaméthasone, la lidocaïne, le minoxidil, l'amphotéricine B, la gentamicine, la rifampicine, la vitamine E et ses esters, le diclofénac, le chlorhydrate de lidocaïne, des alcaloïdes, des hydroxy acides, des antioxydants et des humectants.

**12.** Vésicules selon l'une quelconque des revendications 9 à 11, comprenant en outre des tensioactifs non ioniques ou des co-solvants, et/ou des modificateurs, où les modificateurs sont choisis dans le groupe constitué par le cholestérol, la stéarylamine, l'acide oléique, le dicétylphosphate et le phosphatidylglycérol (PG), de préférence le cholestérol et le phosphatidylglycérol.

**13.** Vésicules selon l'une quelconque des revendications 9 à 12, dans lesquelles ledit hyaluronate (a) a une concentration de 0,01 % à 5 %, de préférence de 0,02 % à 2 %, davantage de préférence de 0,1 % à 1 %, et ledit au moins un phospholipide (b) a une concentration de 0,1 % à 30 %, de préférence de 0,5 % à 25 %, davantage de préférence de 1 % à 15 % ; où lesdits pourcentages sont en poids de hyaluronate ou de phospholipide par rapport au poids de la dispersion vésiculaire.

**14.** Composition pharmaceutique ou cosmétique pour une utilisation topique comprenant les vésicules selon les revendications 9 à 13, comme ingrédient actif, en combinaison avec un ou plusieurs excipients ou adjuvants pharmaceutiquement ou cosmétiquement acceptables.

**15.** Vésicules telles que définies dans l'une quelconque des revendications 9 à 13 ou composition telle que définie dans la revendication 14, pour une utilisation dans le traitement médical de troubles de la peau, tels que la dermatite atopique et séborrhéique ou le psoriasis.

**16.** Utilisation non médicale de vésicules telles que définies dans les revendications 9 à 13 ou d'une composition telle que définie dans la revendication 14, dans le traitement cosmétique de troubles de la peau, tels que la déshydratation, le vieillissement oxydatif, les altérations de cicatrice/chéloïde.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6890901 B, Marriot
- US 2008145415 A
- CN 103520007
- FR 2939317
- WO 2010102770 A

### Non-patent literature cited in the description

- **ARANYA MANOSROI A,B ; PAVEENA WONGTRAKUL C ; JIRADEJ MANOSROI A,B ; HIDEKI SAKAI D,E ; FUMIO SUGAWARA D ; MAKOTO YUASA D,E ; MASAHIKO ABE D,E.** Characterization of vesicles prepared with various non-ionic surfactants mixed with cholesterol. *Received,* 06 February 2003
- **C. SINICO ; M. MANCONI ; M. PEPPI ; D. VALENTI ; F. LAI ; A.M. FADDA.** Liposomes as carriers for dermal delivery of tretinoin: in vitro evaluation of drug permeation and vesicle-skin interaction. *Journal of Controlled Release,* 2005, vol. 103, 123-136
- **MURGIA S ; MELE S ; MONDUZZI M.** Quantitative characterization of phospholipids in milk fat via 31P NMR using a monophasic solvent mixture. *Lipids,* May 2003, vol. 38 (5), 585-91
- **CEVC G.** Lipid vesicles and other colloids as drug carriers on the skin. *Adv Drug Deliv Rev.,* 27 March 2004, vol. 56 (5), 675-711
- **MANCA, M. L. ; ZARU, M. ; MANCONI, M. ; LAI, F. ; VALENTI, D. ; SINICO, C. ; FADDA, A. M.** Glycerosomes: A new tool for effective dermal and transdermal drug delivery. *International Journal of Pharmaceutics,* 2013, vol. 455 (1-2), 66-74